# EUROPÄISCHE PATENTANMELDUNG

(11) **EP 4 198 517 A1**
(43) Veröffentlichungstag der Anmeldung: **21.06.2023**
(21) Anmeldenummer: 21216007.1
(22) Anmeldetag: 20.12.2021
(51) Int. Cl.: G01N 35/00, B01L 7/00, C12Q 1/6851, C12Q 1/686, G16H 40/67

(54) **POINT-OF-CARE REAL-TIME QUANTITATIVE PCR-TESTVORRICHTUNG**

(71) Anmelder: Procomcure Biotech GmbH, 5303 Thalgau (AT)
(72) Erfinder: ÖNDER, Kamil, 5301 Eugendorf / Salzburg (AT)
(74) Vertreter: dompatent von Kreisler Selting Werner - Partnerschaft von Patent- und Rechtsanwälten mbB

(57) **Zusammenfassung**

Point-of-Care Real-time Quantitative PCR-Testvorrichtung, qPCR, mit einem Gehäuse, einem im Gehäuse angeordneten Testmodul (12) zur Aufnahme einer Testprobe und einem mit dem Testmodul (12) verbundene Steuermodul (14), wobei das Steuermodul (14) ausgebildet ist zur Steuerung des Testmoduls (12) zur Durchführung eines qPCR-Tests und Bestimmung eines Testergebnisses, und einem Übertragungsmodul (16), wobei das Übertragungsmodul (16) ausgebildet ist das Testergebnis und/oder Statusinformation der qPCR-Testvorrichtung an mindestens einen Empfänger zu übertragen.

## Beschreibung

Die vorliegende Erfindung betrifft ein Point-of-Care Real-time Quantitative PCR-Testvorrichtung, ein System mit einer solchen qPCR-Testvorrichtung sowie ein Verfahren zur Übermittlung von Testergebnissen von einer solchen qPCR Testvorrichtung an mindestens einem Empfänger.

Heutige Testvorrichtungen und insbesondere quantitative PCR-Testvorrichtungen (qPCR-Testvorrichtungen) befinden sich derzeitig in Großlaboren. Dies erfordert, dass eine entfernte Probeentnahme durchgeführt wird und sodann die Testprobe an das Großlabor übermittelt wird und dort getestet wird auf mögliche Erkrankungen. Aufgrund des physischen Versendens der Testprobe erfordert eine solche Testung eine erhebliche Zeit. Gleichzeitig sind die Testkapazitäten beschränkt auf einige wenige Testlabore, sodass es im Falle eines erhöhten Testaufkommens durch die beschränkte Verfügbarkeit solcher Testvorrichtungen zu weiteren Verzögerungen kommen kann.

Gleichzeitig stellt sich das Problem der Übermittlung der Testergebnisse. Bei bekannten qPCR-Testvorrichtungen erfolgt eine physische Ausgabe der Virenlast der Testprobe, beispielsweise durch einen Drucker. Dieses Testergebnis wird sodann von einem Mitarbeiter händisch in ein System eingegeben und sodann an einen entsprechenden Empfänger übermittelt. Dieses Prozedere ist jedoch ineffizient und fehleranfällig, da Fehler bei der Übertragung der Testergebnisse auftreten können.

Darüber hinaus bestehen bei meldepflichtigen Krankheiten wie beispielsweise Covid-19 zentrale amtliche oder von einem öffentlichen Träger geführte Register in denen Testergebnisse erfasst werden sollen. Somit ist eine zusätzliche Übertragung der Testergebnisse an diese zentrale Stelle erforderlich.

Dies stellt kein Problem dar, solange wenige zertifizierte Testlabore Testergebnisse übermitteln. Um jedoch neue Labore einzurichten, welche hinzukommen, müssen diese Zugang erhalten zu den zentralen Registern. Gleichzeitig muss einem Missbrauch vorgebeugt werden, sodass sowohl die Tests nur von hierzu zugelassenen Testvorrichtungen durchgeführt werden und auch nur die Testergebnisse solcher Testvorrichtungen in den zentralen Registern hinterlegt werden.

Aufgabe der vorliegenden Erfindung ist es daher eine qPCR-Testvorrichtung zu schaffen mit einer verbesserten Bereitstellung der Testergebnisse.

Die Aufgabe wird geschaffen durch eine qPCR-Testvorrichtung gemäß Anspruch 1, ein System gemäß Anspruch 11 sowie ein Verfahren gemäß Anspruch 14.

Die vorliegende Erfindung betrifft ein Point-of-Care Real-time quantitative PCR-Testvorrichtung (qPCR-Testvorrichtung). Hierbei wird die qPCR-Testvorrichtung vorgesehen am Ort der Probenentnahme selbst (point of care), sodass hierdurch bereits die Dauer bis zum Vorliegen eines Testergebnisses reduziert wird, da ein Versenden der Testprobe nicht mehr erforderlich ist. Die qPCR-Testvorrichtung weist ein Gehäuse auf. Dabei ist im Gehäuse ein Testmodul angeordnet zur Aufnahme mindestens einer Testprobe. Mit dem Testmodul ist ein Steuermodul verbunden, wobei das Steuermodul ausgebildet ist zur Steuerung des Testmoduls, sodass durch das Testmodul qPCR-Tests durchgeführt werden zur Bestimmung eines Testergebnisses. Erfindungsgemäß weist die qPCR-Testvorrichtung ein Übertragungsmodul auf, welches mit dem Steuermodul verbunden ist. Dabei ist das Übertragungsmodul ausgebildet, das Testergebnis und/oder Statusinformation der qPCR-Testvorrichtung an mindestens einen Empfänger zu übertragen. Somit ist die qPCR-Testvorrichtung ausgebildet gemäß der vorliegenden Erfindung unmittelbar und ohne manuelle Übertragung des Testergebnisses dieses an mindestens einen Empfänger zu übertragen. Alternativ oder zusätzlich hierzu kann eine Statusinformation der qPCR-Testvorrichtung an den mindestens einen Empfänger mittels des Übertragungsmoduls übertragen werden. Hierdurch kann der Empfänger auf die Funktion der qPCR-Testvorrichtung schließen, wodurch beispielsweise Manipulationen an der qPCR-Testvorrichtung erschwert werden. Beispielsweise kann das Point-of-care qPCR-Testvorrichtung in einer Apotheke, bei einem Hausarzt oder dergleichen aufgestellt sein. An selber Stelle erfolgt sodann die Probenentnahme. Die Probe wird mittels der qPCR-Testvorrichtung getestet. Sodann wird automatisiert das Testergebnis an mindestens einen Empfänger übermittelt. Bei dem Empfänger handelt es sich beispielsweise um eine öffentliche/amtliche Stelle insbesondere zur Registrierung meldepflichtiger Krankheiten, oder eine sonstige Registrierungsstelle/Datenbank zur Registrierung des Testergebnisses. Insbesondere erfolgt hierbei die Übertragung anonymisiert. Alternativ hierzu kann es sich bei dem Empfänger um den Hersteller der qPCR-Testvorrichtung handeln, wobei das Testergebnis und/oder Statusinformation der qPCR-Testvorrichtung übertragen werden. Eine manuelle und damit fehleranfällige Verarbeitung der Testergebnisse ist nicht mehr erforderlich. Auch eine automatisierte Hinterlegung in eine zentrale Datenbank oder einem zentralen Register ist hierdurch automatisiert möglich, wodurch mehr Testergebnisse in kürzerer Zeit verarbeitet werden können. Weiterhin ist durch die vorliegende Erfindung die Möglichkeit der Manipulation der Testergebnisse reduziert, da kein Zwischenschritt erfolgt, bei dem Mitarbeiter involviert sind.

Vorzugsweise ist das Testmodul ausgebildet zur Detektion von COVID, Influenza und/oder RSV (Respiratorische Synzytial-Virus-Infektionen). Somit kann die qPCR-Testvorrichtung ausgebildet sein zur dedizierten Detektion eines Erregers. Alternativ ist die qPCR-Testvorrichtung ausgebildet, so dass durch diese unterschiedliche Erreger detektiert werden können.

Vorzugsweise ist das Testmodul ausgebildet mehrere Testproben aufzunehmen zur Testung. Hierdurch können durch das Testmodul mehrere Testproben gleichzeitig getestet werden auf eine mögliche Virenlast. Hierdurch wird die Verarbeitungsgeschwindigkeit weiter erhöht und Testergebnisse stehen schneller zu Verfügung.

Vorzugsweise kann ein Testergebnis übertragen werden. Alternativ können eine Vielzahl von Testergebnissen gleichzeitig übertragen werden. Ebenso kann die übertragenen Statusinformation einen einzelnen Status der qPCR-Testvorrichtung enthalten, welcher sodann übertragen wird. Alternativ hierzu enthält die Statusinformation eine Vielzahl unterschiedlicher Informationen über den Status der qPCR-Testvorrichtung, welche vorzugsweise gleichzeitig übertragen werden. Vorzugsweise ist das Übertragungsmodul ausgebildet eine gesicherte Verbindung mit dem mindestens einen Empfänger aufzubauen. Hierbei wird beispielsweise SSL verwendet zwischen der qPCR-Testvorrichtung und dem Empfänger, wobei mittels Zertifikataustausch eine sichere Verbindung aufgebaut werden kann. Hierdurch werden die Manipulationsmöglichkeiten weiter reduziert und die Sicherheit der Daten erhöht.

Vorzugsweise ist das Übertragungsmodul ausgebildet das Testergebnis an mehrere Empfänger zu übermitteln. Alternativ oder zusätzlich hierzu ist das Übertragungsmodul ausgebildet Statusinformation an mehrere Empfänger zu übermitteln. Somit ist es möglich, dass durch die qPCR-Testvorrichtung das Testergebnis nicht lediglich an einen Empfänger übertragen wird, sondern die gleichzeitige Bereitstellung des Testergebnisses und/oder der Statusinformation an mehrere Empfänger ermöglicht wird. Insbesondere im Rahmen eines Gesundheitssystems, welches an der Überwachung einer Ausbreitung möglicher Krankheiten interessiert ist, kann es erforderlich sein Testergebnisse, insbesondere anonymisiert, an mehrere Empfänger zu übermitteln, um beispielsweise hieraus Statistiken, Vorhersagen oder Informationen an die Bürger über die Ausbreitung der Krankheit und/oder die Belastung des Gesundheitssystems bereitzustellen. Eine manuelle Weitergabe der Testergebnisse innerhalb dieser Informationssysteme, welche zu Verzögerungen und Fehlern führen würde, ist nicht mehr erforderlich.

Vorzugsweise umfasst die Statusinformation eines oder mehreres von einem Verbindungszustand der qPCR-Testvorrichtung, einen Fehlerindikator und einen Testdurchführungsindikator. Mittels des Verbindungszustands kann ermittelt werden, ob die qPCR-Testvorrichtung online ist und in der Lage ist Testergebnisse mittelts des Übertragungsmoduls zu übertragen. Mittels des Fehlerindikators können Fehlerzustände der qPCR-Testvorrichtung angezeigt werden. Insbesondere wenn die Statusinformation an den Hersteller der qPCR-Testvorrichtungen übermittelt werden, kann dieser hieraus mögliche Reparaturen bereits ableiten und entsprechend vorbereite. Gleichzeitig wird durch den Fehlerindikator sichergestellt, dass nur korrekte Testergebnisse weitergeleitet werden. Zeigt somit der Fehlerindikator einen Fehler an, welcher insbesondere einen Einfluss auf das Testergebnis haben könnte, so werden weitere Testungen durch die qPCR-Testvorrichtung ausgeschlossen oder deren Testergebnisse verworfen. Mittels des Testdurchführungsindikators wird angezeigt, ob die qPCR-Testvorrichtung derzeit einen Test durchführt. Hierdurch kann die Aktivität der qPCR-Testvorrichtung weiter überwacht werden. Insbesondere kann hierdurch ein kausaler Zusammenhang zwischen der Durchführung eines Tests und der Bereitstellung von Testergebnisse hergestellt werden, sodass das Bereitstellen manipulierter Testergebnisse weiter erschwert wird.

Vorzugsweise umfassen die Testergebnisse eines oder mehreres von einem Testindikator, einer Virenlast, einer Personen-ID und einem Überprüfungsindikator. Der Testindikator liefert einen Hinweis auf eine Erkrankung des Probanden. Dabei kann der Testindikator insbesondere binär ausgebildet sein und stellt somit das Ergebnis der Auswertung des qPCR-Tests dar. Weiterhin kann das Testergebnis eine Virenlast enthalten, welche durch den qPCR-Test ermittelt wurde. Weiterhin kann das Testergebnis eine Personen-ID enthalten. Insbesondere handelt es sich bei der Personen-ID um eine anonymisierte Identifikation des Probanden. Diese Personen-ID kann beispielsweise als Barcode zur Verfügung gestellt werden, mittels dem eine Zuordnung der Proben erfolgt. Insbesondere umfasst die Personen-ID keine personenbezogenen Informationen (wie beispielsweise Name, Anschrift, Passnummer oder dergleichen) und wird beispielsweise von dem Testgerät erstellt. Insbesondere enthält die Personen-ID eine Geräte-ID der qPCR-Testvorrichtung. Hierdurch wird sichergestellt, dass die Personen-ID der jeweiligen qPCR-Testvorrichtung eindeutig ist und eine Kollision von Personen-IDs unterschiedlicher Testvorrichtungen ausgeschlossen wird. Alternativ erfolgt eine zentrale Vergabe der Personen-ID, um die Eindeutigkeit der Personen-ID sicherzustellen. Weiterhin kann das Testergebnis einen Überprüfungsindikator aufweisen. Mittels des Überprüfungsindikators wird angezeigt, ob es sich bei der Probe um die Probe eines Menschen handelt. Hierdurch werden Manipulationen der Probe insbesondere bei der Probenentnahme verhindert.

Vorzugsweise wird die Statusinformation periodisch an den mindestens einen Empfänger übertragen insbesondere mittels http-Anfrage bzw. http-request. Hierdurch wird eine einfacher Übertragungsweg der Statusinformation an den mindestens einen Empfänger bereitgestellt. Dabei kann die Statusinformation beispielsweise einmal pro Stunde, einmal pro Minute oder alle 10 Sekunden an den mindestens einen Empfänger übertragen werden. Die vorliegende Erfindung ist nicht beschränkt auf eine bestimmte Häufigkeit der Übertragung der Statusinformation, solange diese Häufigkeit hoch genug ist, um eine sichere Überwachung der qPCR-Testvorrichtung und deren Funktionalität sicherzustellen.

Vorzugsweise ist eine Zugangsinformation eines Empfängers im Übertragungsmodul oder dem Steuermodul hinterlegt. Alternativ hierzu weist die qPCR-Testvorrichtung ein Speichermodul auf, wobei das Speichermodul mit dem Steuermodul und/oder dem Übertragungsmodul verbunden ist und ausgebildet ist Zugangsdaten eines Empfängers zu speichern. Diese Zugangsinformation kann beispielsweise ein Passwort und/oder eine Geräte-ID umfassen. Mittels den Zugangsinformationen ist eine Übertragung von Testergebnissen und/oder Statusinformationen an den Empfänger möglich. Handelt es sich bei dem Empfänger beispielsweise um eine öffentliche/amtliche Stelle zur Registrierung der Testergebnisse bzw. eine Datenbank, ist der Zugriff auf diese öffentliche Stelle bzw. die Datenbank geschützt. Lediglich bei Vorliegen der Zugangsinformationen können Testergebnisse und/oder Statusinformationen an die zentrale Stelle bzw. den Empfänger übertragen werden. Stimmen die Zugangsinformationen nicht überein mit den bei dem Empfänger hinterlegten Zugangsinformationen für die entsprechende qPCR-Testvorrichtung, ist eine Übertragung der Testergebnisse und/oder Statusinformationen an diesen Empfänger nicht möglich.

Vorzugsweise ist das Übertagungsmodul ausgebildet die Zugangsinformationen für einen zweiten Empfänger an einen ersten Empfänger zu übertragen. Somit können beispielsweise Zugangsinformationen zusammen mit den Testergebnissen und/oder Statusinformationen von der qPCR-Testvorrichtung unmittelbar an den ersten Empfänger automatisiert übertragen werden. Sodann erfolgt eine Weiterübertragung an den zweiten Empfänger, wobei die Zugangsinformationen genutzt werden, um die Testergebnisse an den zweiten Empfänger weiterzuleiten bzw. diese dort zu hinterlegen wie vorstehend beschrieben. Hierbei werden die Zugangsinformationen verschlüsselt, insbesondere als JASON Web Token, von der qPCR-Testvorrichtung an den ersten Empfänger übertragen, sodass der erste Empfänger keinerlei Kenntnis über die Zugangsinformationen hat. Eine Entschlüsselung durch den ersten Empfänger ist hierbei nicht möglich. Erst durch Empfang der Zugangsinformation bzw. des JASON Web Token durch den zweiten Empfänger kann dieser die verschlüsselte Zugangsinformation entschlüsseln und als Zugangsinformation verwendet werden zur Weiterverarbeitung/Hinterlegung des Testergebnisses. Somit die übertragenen Testergebnisse und/oder Statusinformationen vom zweiten Empfänger empfangen und weiterverarbeitet. Hierdurch ist eine flexible Ausgestaltung des Informationsflusses von der qPCR-Testvorrichtung an die mehreren Empfänger möglich. So kann beispielsweise zunächst insbesondere mittels einer gesicherten SSL Verbindung eine Übertragung der Testergebnisse und/oder Statusinformationen von der qPCR-Testvorrichtung an den ersten Empfänger erfolgen, wobei es sich bei dem ersten Empfänger beispielsweis um den Hersteller der qPCR-Testvorrichtung handelt oder einen Service-Provider. Von diesem werden sodann die Testergebnisse und/oder Statusinformationen an einen zweiten Empfänger weitergeleitet. Bei dem zweiten Empfänger handelt es sich beispielsweise um eine öffentliche/amtliche Stelle wie beispielsweise ein Gesundheitsamt, einen Apothekenverband, einen Ärzteverband oder dergleichen, durch die eine Weiterverarbeitung der Testergebnisse erfolgen soll. Die entsprechenden Zugangsdaten, welchen den Zugriff auf den zweiten Empfänger zur Hinterlegung der Testergebnisse enthalten, werden dabei von der qPCR-Testvorrichtung insbesondere in verschlüsselter Form beispielsweise als JASON Web Token mitübertragen. Die Kenntnis der Zugangsdaten durch den Hersteller als ersten Empfänger ist somit gerade ausgeschlossen, da eine Entschlüsselung der Zugangsinformation durch den ersten Empfänger nicht erfolgt oder ausgeschlossen ist. Dennoch kann eine Weiterleitung an den jeweils zweiten Empfänger erfolgen. Sollen weitere oder andere zweite Empfänger die Testergebnisse ebenfalls zur Verfügung gestellt werden, ist es nicht mehr erforderlich einzelne qPCR-Testvorrichtungen umzukonfigurieren. Vielmehr kann sodann eine Weiterleitung durch den ersten Empfänger, wie beispielsweise den Hersteller oder einen Service-Provider, unmittelbar und zentral an diese neuen zweiten Empfänger erfolgen. Hierdurch wird der Informationsfluss flexibel und die Anpassung, an wen die jeweiligen Testergebnisse und/oder Statusinformationen weitergeleitet werden, ist auf einfache Weise möglich.

Weiterhin betrifft die vorliegende Erfindung ein System mit einer qPCR-Testvorrichtung wie vorstehend beschrieben sowie mindestens einem ersten Empfänger.

Vorzugsweise ist der erste Empfänger ausgebildet zur Weiterleitung von Testergebnissen an einen zweiten Empfänger.

Vorzugsweise handelt es sich bei dem ersten Empfänger um einen Hersteller der qPCR-Testvorrichtung oder um einen Service-Provider. Insbesondere handelt es sich bei dem zweiten Empfänger um eine öffentliche/amtliche Hinterlegungsstelle wie beispielsweise ein Gesundheitsamt, ein Ärzteverband, ein Apothekerverband oder dergleichen.

Weiterhin betrifft die vorliegende Erfindung ein Verfahren zur Übermittlung von Testergebnisse einer Point-of-Care Real-time Quantitative PCR-Testvorrichtung (qPCR-Testvorrichtung) mit den Schritten:
- Erzeugung eines Testergebnisses und/oder einer Statusinformation und
- Übertragung des Testergebnisses und/oder Statusinformations an mindestens einen Empfänger.

Vorzugsweise ist die qPCR-Testvorrichtung ausgebildet wie vorstehend beschrieben.

Vorzugsweise erfolgt die Übertragung drahtgebunden oder drahtlos mittels bekannter Übertragungsarten, beispielsweise über das Internet mittels LAN, WLAN, einem GSM Standard, oder einer anderen drahtlosen Übertragungstechnik wie beispielsweise Bluetooth, ZigBee, oder dergleichen. Dabei kann die Übertragung über mehrere Knoten oder Accesspoints erfolgen. Insbesondere ist die vorliegende Erfindung nicht beschränkt auf die spezielle Art der Übertragung der Testergebnisse und/oder Statusinformationen.

Vorzugsweise erfolgt die Übertragung verschlüsselt bzw. gesichert insbesondere mittels SSL.

Vorzugsweise werden die Statusinformationen periodisch an den mindestens einen Empfänger übermittelt.

Vorzugsweise wird das Testergebnis an mehrere Empfänger übertragen. Dabei kann die Übertragung ausgehend von der qPCR-Testvorrichtung unmittelbar an mehrere Empfänger erfolgen. Alternativ hierzu erfolgt die Weiterleitung der Testergebnisse durch einer der Empfänger.

Vorzugsweise erfolgt die Übermittlung der Statusinformationen nur an einen Empfänger.

Vorzugsweise werden Zugangsinformationen zum Zugang zu einem Empfänger auf qPCR-Testvorrichtung hinterlegt. Somit können die Testergebnisse zusammen mit den Zugangsinformationen an den mindestens einen Empfänger versendet werden. Dabei sind die Zugangsinformationen erforderlich um eine Hinterlegung der Testergebnisse und/oder Statusinformationen bei dem mindestens einen Empfänger zu erreichen.

Vorzugsweise werden die Zugangsinformationen verschlüsselt insbesondere mittels JASON Web Token zusammen mit den Testergebnissen und/oder Statusinformationen an einen ersten Empfänger übertragen. Hierbei kann beispielsweise die Übertragung mittels einer SSL Sicherung erfolgen. Sodann erfolgt eine Weiterleitung der Testergebnisse mittels der Zugangsinformationen an einen zweiten Empfänger, wobei die Zugangsinformationen erforderlich sind zur Hinterlegung der Testergebnisse bei dem zweiten Empfänger. Dabei kann es sich bei dem ersten Empfänger beispielsweise um den Hersteller der qPCR-Testvorrichtung handeln oder einen Service-Provider, wobei es sich bei dem zweiten Empfänger beispielsweise um eine öffentliche/amtliche Stelle wie beispielsweise einem Gesundheitsamt, einem Apothekenverband und/oder Ärzteverband handelt. Somit kann die Hinterlegung der Testergebnisse zur weiteren Verwendung durch den zweiten Empfänger lediglich erfolgen bei Vorliegen der Zugangsinformationen. Dabei erfolgt die Weiterleitung der Zugangsinformationen ohne, dass der erste Empfänger Kenntnis hierüber erlangt. Eine Entschlüsselung der Zugangsinformationen durch den ersten Empfänger ist nicht möglich. Somit ist eine Hinterlegung manipulierter Testergebnisse beispielsweise durch den ersten Empfänger nicht möglich, da dieser keine Kenntnis über die Zugangsinformationen hat. Auch ein ungewolltes Auslesen von Testergebnissen durch den ersten Empfänger ist nicht möglich, da dieser keinen Zugriff auf den zweiten Empfänger hat.

Vorzugsweise werden durch den mindestens einen Empfänger Verbrauch von Testproben oder weiterem Testmaterial ermittelt aus der Statusinformation und/oder Planungsinformationen, welche von der qPCR-Testvorrichtung und/oder dem Betreiber der qPCR-Testvorrichtung an den mindestens einen Empfänger übertragen werden. Bei dem Planungsinformationen kann es sich beispielsweise um Informationen hinsichtlich der zukünftig anstehenden durchzuführenden Tests handeln. Diese können beispielsweise automatisiert von dem Betreiber der qPCR-Testvorrichtung an den ersten Empfänger weitergeleitet werden. Insbesondere handelt es sich bei dem Empfänger um den Hersteller der qPCR-Testvorrichtung bzw. einen Service-Provider. Unterschreitet eine Anzahl an verfügbaren Testproben oder die Verfügbarkeit weiteren Testmaterials einen vorgegebenen Grenzwert, wird ein Signal ausgelöst. Bei diesem Signal kann es sich um ein optisches oder akustisches Signal handeln, welches bei dem mindestens einen Empfänger ausgegeben wird. Alternativ handelt es sich bei dem Signal um ein Steuerungssignal, welches eine automatisierte Bereitstellung weiterer Testproben oder weiteren Testmaterials an den Betreiber der qPCR-Testvorrichtung auslöst. Hierbei kann insbesondere anhand der Statusinformation und/oder Planungsinformation die voraussichtliche Menge, erforderlicher Testproben und/oder Testmaterial ermittelt werden und somit die Anzahl der nachzuliefernden Testproben oder den Umfang des nachzuliefernden Testmaterials angepasst werden.

Vorzugsweise ist die qPCR-Testvorrichtung weitergebildet anhand der Merkmale des vorstehend beschriebenen Verfahrens.

Nachfolgend wird die Erfindung anhand bevorzugter Ausführungsformen unter Bezugnahme der beigefügten Figuren näher erläutert.

Es zeigen:
Figur 1 eine schematische Darstellung der qPCR-Testvorrichtung gemäß der vorliegenden Erfindung,
Figur 2 ein schematisches Ablaufdiagramm gemäß dem Verfahren der vorliegenden Erfindung,
Figur 3 eine schematische Darstellung des Systems gemäß der vorliegenden Erfindung in einer Ausführungsform und
Figur 4 eine schematische Darstellung eines Systems gemäß der vorliegenden Erfindung in einer weiteren Ausführungsform.

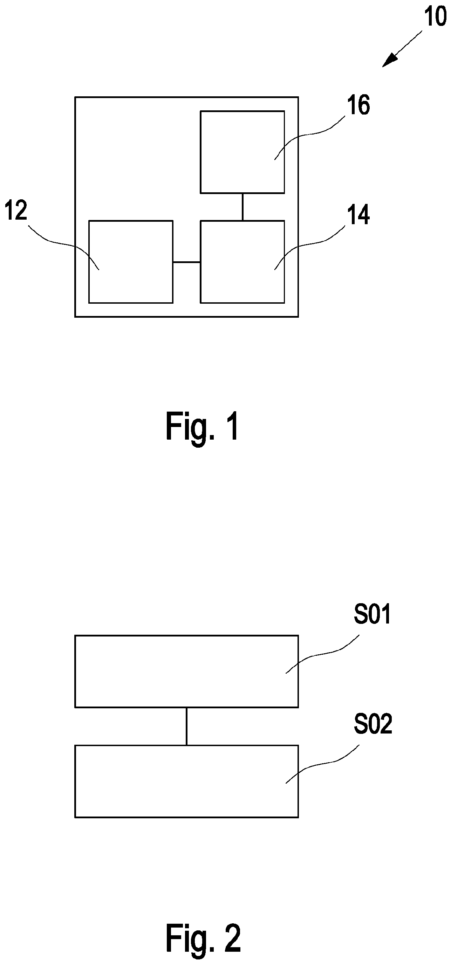

Figur 1 zeigt schematisch ein Point-of-Care Real-time Quantitative PCR-Testvorrichtung (qPCR-Testvorrichtung) 10 mit einem Testmodul 12 zur Aufnahme einer oder mehrerer Testproben. Mit dem Testmodul 12 ist ein Steuermodul 14 verbunden. Dabei wird mittels des Steuermoduls 14 das Testmodul 12 gesteuert zur Durchführung eines oder mehrerer qPCR-Tests zur Bestimmung eines Testergebnisses. Mittels des Testmoduls kann für jede Testprobe ein qPCR-Testergebnis ermittelt werden. Dabei kann das Steuermodul 14 eine Eingabevorrichtungen aufweisen wie beispielsweise eine Tastatur oder einen Touchscreen. Weiterhin kann das Steuermodul eine Ausgabevorrichtung aufweisen wie beispielsweise ein Display oder dergleichen. Insbesondere weist das Steuermodul eine Steuersoftware auf. Diese stellt ein Graphical-User-Interface (GUI - Benutzeroberfläche) bereit, welches auf dem Display des Steuermoduls 14 angezeigt. Parameter, welche von dem Benutzer der qPCR-Testvorrichtung 10 eingegeben werden mittels der Eingabevorrichtung, insbesondere über den Touchscreen der qPCR-Testvorrichtung 10, werden sodann in Steuerparameter übersetzt und an das Betriebssystem der qPCR-Testvorrichtung 10 weitergeleitet zur Steuerung des Testmoduls 12. Hierbei wird insbesondere durch die Software und/oder das Graphical-User-Interface bereitgestellt durch die Software ein Zugriff auf die Funktionen des Betriebssystems der qPCR-Testvorrichtung unterbunden, sodass eine ungewollte Ansteuerung der qPCR-Testvorrichtung 10 und/oder eine Manipulation der qPCR-Testvorrichtung 10 ausgeschlossen wird. So werden beispielsweise durch die Software nur solche Funktionen bereitgestellt, welche auch durch den Benutzer genutzt werden sollen. Darüber hinaus kann die Software einen insbesondere passwortgeschützten Servicezugang bereitstellen, welcher sodann Zugriff auf die Funktionen des Betriebssystems der qPCR-Testvorrichtung 10 bereitstellt.

Mit dem Steuermodul 14 ist ein Übertagungsmodul 16 verbunden. Das Übertagungsmodul 16 ist dabei ausgebildet Testergebnisse und/oder Statusinformationen der qPCR-Testvorrichtung 10 und insbesondere des Testmoduls 12 an mindestens einen Empfänger zu übertragen. Dabei kann das Übertragungsmodul 16 eine LAN-, WLAN-, WIFI-Schnittstelle aufweisen oder eine Schnittstelle eines GSM-Standards oder einer sonstigen Funkübertragungstechnik wie beispielsweise Bluetooth, ZigBee oder dergleichen. Die vorliegende Erfindung ist nicht auf die spezielle Art der Übertragung beschränkt.

Mittels des Übertragungsmodul 16 erfolgt somit eine automatisierte Weiterleitung der der Testergebnisse an einen Empfänger.

Ebenfalls werden insbesondere durch das Steuermodul 14 Statusinformation bereitgestellt. Diese können ebenfalls mittels dem Übertragungsmodul 26 an den mindestens einen Empfänger übertragen werden.

Das Verfahren ist in Figur 2 schematisch dargestellt. Im Schritt S01 wird ein Testergebnis ermittelt. Dieses Testergebnis wird insbesondere ermittelt mittels einer Testvorrichtung 10 wie in Figur 1 beschrieben. Sodann erfolgt eine insbesondere automatisierte Weiterleitung des Testergebnisses im Schritt S02. Insbesondere erfolgt keine manuelle Verarbeitung der Testergebnisse und/oder Statusinformationen vor der Weiterleitung an den mindestens einen Empfänger. Hierzu ist wie vorstehend beschrieben, die verwendete qPCR-Testvorrichtung 10 selbst ausgebildet die Testergebnisse an mindestens einen und insbesondere mehrere Empfänger zu übertragen.

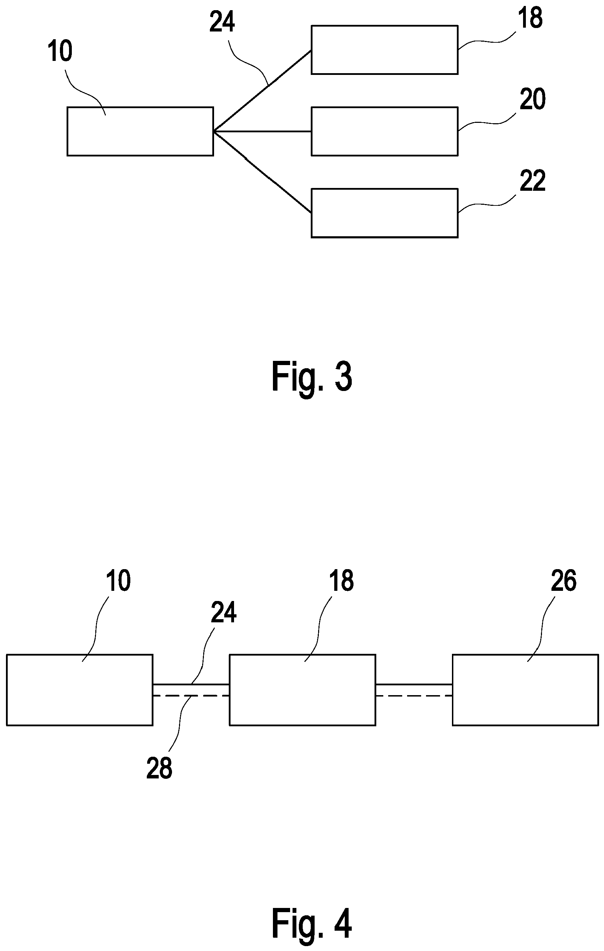

Dabei kann es sich bei den Empfängern um mehrere Empfänger handeln, wie in Figur 3 dargestellt, welche über Verbindungen 24 unmittelbar Statusinformation und/oder Testergebnisse von der qPCR-Testvorrichtung 10 übermittelt bekommen. So kann es sich beispielsweise bei einem Empfänger 18 um den Hersteller der qPCR-Testvorrichtung handeln. Dieser erhält insbesondere die Testergebnisse, welche anonymisiert sind und Statusinformationen über die Funktionalität der qPCR-Testvorrichtung 10. Insbesondere erhält ausschließlich der Hersteller 18 solche Statusinformation. Ein weiterer Empfänger 20 kann beispielsweise eine öffentliche Stelle oder amtliche Stelle zur Hinterlegung von Gesundheitsdaten sein. Beispielsweise handelt es sich bei dem weiteren Empfänger 20 um ein Gesundheitsamt, welches die Testergebnisse empfängt. Insbesondere ist es hierbei nicht erforderlich, dass die Testergebnisse anonymisiert sind. Dies ist jedoch möglich. Wird beispielsweise eine zentrale Hinterlegungsstelle für bestimmte meldepflichtigen Krankheiten wie insbesondere COVID geschaffen, kann somit durch die qPCR-Testvorrichtung der vorliegenden Erfindung eine automatische Hinterlegung von Gesundheitsdaten in diese Datenbank erfolgen. Manipulationsmöglichkeiten sind hierbei reduziert, da keine manuelle Übertragung/Verarbeitung der Testergebnisse, manuelle Weiterleitung der Testergebnisse und/oder manuelle Registrierung der Testergebnisse in der entsprechenden Datenbank des weiteren Empfängers 20 erfolgt.

Ein weiterer Empfänger 22 kann beispielsweise der Betreiber der qPCR-Testvorrichtung 10 sein und/oder ein Verband wie beispielsweise ein Apothekerverband und/oder ein Ärzteverband. Hierbei können die Testergebnisse anonymisiert oder nicht anonymisiert weitergeleitet werden.

Insbesondere erfolgt dabei die Übertagung der Testergebnisse und/oder Statusinformationen von der qPCR-Testvorrichtung an die jeweiligen Empfänger 18, 20, 22 über die Verbindung 24 in verschlüsselter und gesicherter Form. Insbesondere erfolgt eine SSL Sicherung der Verbindung 24 durch Austausch eines geeigneten Zertifikats.

Im Folgenden wird Bezug genommen auf Figur 4. In einer Ausführungsform erfolgt zunächst eine Übertragung der erfassten Testergebnisse und/oder Statusinformationen von der qPCR-Testvorrichtung 10 über eine Verbindung 24 an einen ersten Empfänger 18. Bei dem ersten Empfänger 18 kann es sich beispielsweise um einen Hersteller oder einen Service-Provider handeln. Sodann werden die Testergebnisse und/oder Statusinformationen von dem ersten Empfänger 18 an einen zweiten Empfänger 26 übertragen. Dabei können nur die Testergebnisse von dem ersten Empfänger 18 auf den zweiten Empfänger 26 übertragen werden, nur die Statusinformation von dem ersten Empfänger 18 auf den zweiten Empfänger 26 übertragen werden oder eine Kombination aus Testergebnissen und Statusinformation. Insbesondere ist es erforderlich zur Hinterlegung der Testergebnisse und/oder Statusinformation bei dem zweiten Empfänger 26 eine Zugangsinformation zu verwenden wie beispielsweise eine Nutzerkennung und/oder ein Passwort. Hierdurch ist der Zugang zu dem zweiten Empfänger 26, der Datenbank des zweiten Empfängers 26 oder dergleichen geschützt. Hierzu ist die Zugangsinformation auf der qPCR-Testvorrichtung 10 hinterlegt. Eine weitere Hinterlegung der Zugangsinformation an anderen/weiteren Stellen ist aus Gründen der Sicherheit nicht gewollt. Dabei ist gewünscht, dass der erste Empfänger 18 keine Kenntnis über diese Zugangsinformationen erhält, um eine Hinterlegung manipulierter Testergebnisse beim zweiten Empfänger 26 oder Auslesen weiterer Testergebnisse vom zweiten Empfänger 26 durch den ersten Empfänger 18 zu verhindern. Hierzu erfolgt insbesondere eine Weiterleitung der Zugangsinformation 28 zusammen mit dem Testergebnis und/oder der Statusinformation, wobei die Zugangsinformation in verschlüsselter Form von der qPCR-Testvorrichtung 10 an den ersten Empfänger 18 übertragen werden und sodann zusammen mit dem Testergebnis und/oder der Statusinformation an den zweiten Empfänger 26 weitergeleitet werden. Dieser kann die Verschlüsselung der Zugangsinformationen entfernen und die Zugangsinformationen nutzen, um eine Hinterlegung der Testergebnisse und/oder Statusinformationen beim zweiten Empfänger 26 zu gewährleisten. Dabei kann die Weiterleitung der Zugangsinformationen beispielsweise als JASON Web Token erfolgen. Hierdurch wird sichergestellt, dass der erste Empfänger 18 keine Kenntnis über die Zugangsinformation erhält.

Gleichzeitig wird durch die in der Figur 4 gezeigte Informationsstruktur sichergestellt, dass eine flexible Weiterleitung der Testergebnisse an relevante Empfänger gewährleistet ist. Wird beispielsweise ein weiterer zweiter Empfänger hinzugefügt, an den Testergebnisse und/oder Statusinformationen ebenfalls weitergeleitet werden oder ändert sich der zweite Empfänger 26, muss dies nicht in jeder qPCR-Testvorrichtung 10 geändert und hinterlegt werden. Vielmehr reicht eine zentrale Änderung beim ersten Empfänger 18 als Hersteller oder Service-Provider.

Somit wird durch die vorliegende Erfindung ein System und Verfahren geschaffen, mit dem Gesundheitsdaten effizient erfasst und weitergeleitet werden können. Gleichzeitig wird die Integrität der Daten gewährleistet. Insbesondere wird die Sicherheit der Entitäten des Systems untereinander aufrechterhalten durch lokale Hinterlegung der Zugangsdaten ohne eine zentrale Hinterlegung. Gleichzeitig bietet die vorliegende Erfindung ausreichend Flexibilität, um das System an unterschiedliche gesetzliche Vorgaben anzupassen, beispielsweise bei der Erfassung einer pandemischen Lage.

## Patentansprüche

1. Point-of-Care Real-time Quantitative PCR-Testvorrichtung, qPCR, mit einem Gehäuse,
einem im Gehäuse angeordneten Testmodul (12) zur Aufnahme einer Testprobe und
einem mit dem Testmodul (12) verbundene Steuermodul (14), wobei das Steuermodul (14) ausgebildet ist zur Steuerung des Testmoduls (12) zur Durchführung eines qPCR-Tests und Bestimmung eines Testergebnisses, und
einem Übertragungsmodul (16), wobei das Übertragungsmodul (16) ausgebildet ist das Testergebnis und/oder Statusinformation der qPCR-Testvorrichtung an mindestens einen Empfänger zu übertragen.

2. qPCR-Testvorrichtung nach Anspruch 1, **dadurch gekennzeichnet, dass** das Testmodul ausgebildet ist COVID, Influenza und/oder RSV zu detektieren.

3. qPCR-Testvorrichtung nach Anspruch 1 oder 2, **dadurch kennzeichnet, dass** das Testmodul ausgebildet ist mehrere Testproben aufzunehmen zur Testung.

4. qPCR-Testvorrichtung nach einem der Ansprüche 1 bis 3, **dadurch gekennzeichnet, dass** das Übertragungsmodul ausgebildet ist eine gesicherte Verbindung insbesondere mittels SSL mit dem mindestens einen Empfänger aufzubauen.

5. qPCR-Testvorrichtung nach einem der Ansprüche 1 bis 4, **dadurch gekennzeichnet, dass** das Übertragungsmodul ausgebildet ist das Testergebnis und/oder Statusinformation an mehrere Empfänger zu übermitteln.

6. qPCR-Testvorrichtung nach einem der Ansprüche 1 bis 5, **dadurch gekennzeichnet, dass** die Statusinformation eines oder mehreres von einem Verbindungszustand der qPCR-Testvorrichtung, einem Fehlerindikator, einen Testdurchführungsindikator.

7. qPCR-Testvorrichtung nach einem der Ansprüche 1 bis 6, bei welchem das Testergebnis eines oder mehreres enthalten von einem Testindikator, einer Virenlast, einer Personen-ID und einer Überprüfungsindikator.

8. qPCR-Testvorrichtung nach einem der Ansprüche 1 bis 7, **dadurch gekennzeichnet, dass** die Statusinformation periodisch an den mindestens einen Empfänger übertragen wird insbesondere mittels http-Anfrage.

9. qPCR-Testvorrichtung nach einem der Ansprüche 1 bis 8, **dadurch gekennzeichnet, dass** eine Zugangsinformation mindestens eines Empfängers im Übertragungsmodul oder dem Steuermodul hinterlegt ist.

10. qPCR-Testvorrichtung nach Anspruch 9, **dadurch gekennzeichnet, dass** das Übertragungsmodul ausgebildet ist die insbesondere verschlüsselten Zugangsinformation für einen zweiten Empfänger an einen ersten Empfänger zu übertragen insbesondere zur Weiterleitung an den zweiten Empfänger.

11. System mit einer qPCR-Testvorrichtung nach einem der Ansprüche 1 bis 10 und mindestens einem ersten Empfänger.

12. System nach Anspruch 11, **dadurch gekennzeichnet, dass** der erste Empfänger ausgebildet ist zur Weiterleitung von Testergebnissen an einen zweiten Empfänger.

13. System nach Anspruch 11 oder 12, bei dem es sich bei dem ersten Empfänger um einen Hersteller der qPCR-Testvorrichtung handelt und bei dem zweiten Empfänger um eine öffentliche Hinterlegungsstelle.

14. Verfahren zur Übermittlung von Testergebnissen einer Point-of-Care Quantitative PCR-Testvorrichtung, qPCR, insbesondere nach einem der Ansprüche 1 bis 10, an mindestens einen Empfänger, mit den Schritten:
Erzeugen eines Testergebnisses und/oder einer Statusinformation;
Übertragen des Testergebnisses und/oder der Statusinformation an mindestens einen Empfänger.

15. Verfahren nach Anspruch 14, bei welchem die Übertragung verschlüsselt erfolgt, insbesondere mittels SSL.

16. Verfahren nach Anspruch 14 oder 15, bei welchem die Statusinformation periodisch an den mindestens einen Empfänger übermittelt werden.

17. Verfahren nach einem der Ansprüche 14 bis 15, bei welchem das Testergebnis an mehrere Empfänger übertragen wird.

18. Verfahren nach einem der Ansprüche 14 bis 17, bei welchem eine Zugangsinformation zum Zugang zu mindestens einem Empfänger auf der qPCR-Testvorrichtung hinterlegt wird.

19. Verfahren nach einem der Ansprüche 14 bis 18, bei welchem eine Zugangsinformation verschlüsselt insbesondere mittels JASON Web Token zusammen mit Testergebnissen an einen ersten Empfänger übertragen werden, wobei der erste Empfänger mittels der Zugangsinformation die Testergebnisse an einen zweiten Empfänger weiterleitet.

20. Verfahren nach einem der Ansprüche 14 bis 19, bei welchem insbesondere Planungsinformationen an den mindestens einen Empfänger übermittelt werden, wobei der Empfänger einen Verbrauch von Testproben ermittelt aus den Statusinformationen und/oder Planungsinformationen, wobei bei Unterschreiten einer Anzahl an verfügbaren Testproben einen vorgegebenen Grenzwert ein Signal ausgelöst wird.
